(19) Europäisches Patentamt
European Patent Office
Office européen des brevets

(11) Veröffentlichungsnummer: **0 303 180**
**A1**

(12) # EUROPÄISCHE PATENTANMELDUNG

(21) Anmeldenummer: **88112678.3**

(22) Anmeldetag: **04.08.88**

(51) Int. Cl.⁴: **C07D 279/16 , C07D 417/12 , A61K 31/54**

(30) Priorität: **12.08.87 DE 3726759**

(43) Veröffentlichungstag der Anmeldung: **15.02.89 Patentblatt 89/07**

(84) Benannte Vertragsstaaten: **CH DE FR GB IT LI NL**

(71) Anmelder: **HOECHST AKTIENGESELLSCHAFT**
**Postfach 80 03 20**
**D-6230 Frankfurt am Main 80(DE)**

(72) Erfinder: **Lerch, Ulrich, Dr.**
**Vorderwart 24**
**D-6238 Hofheim am Taunus(DE)**
Erfinder: **Henning, Rainer, Dr.**
**Rotenhofstrasse 31**
**D-6234 Hattersheim am Main(DE)**
Erfinder: **Urbach, Hansjörg, Dr.**
**Le Lavandoustrasse 41**
**D-6242 Kronberg/Taunus(DE)**
Erfinder: **Kaiser, Joachim, Dr.**
**Fichtestrasse 12**
**D-6000 Frankfurt am Main(DE)**

(54) **Benzothiazinon-oxide, Verfahren zu ihrer Herstellung, sie enthaltende Arzneimittel und deren Verwendung sowie Zwischenprodukte zu ihrer Herstellung.**

(57) Verbindungen I

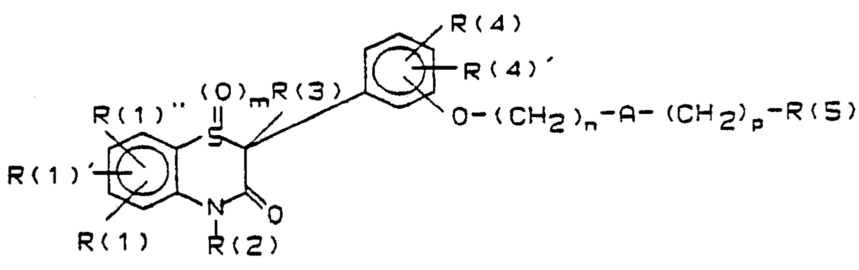

(I)

worin R(1) und R(4) gleich H, Alkyl, Alkoxy, Hal, $CF_3$, $NO_2$, OH, Acetamido, Amino;
R(2) gleich H, Alk(en)yl, Phenylalkyl (ggf. subst.);
R(3) gleich H, (CycloAlk(en)yl-(alkyl), Phenyl,Phenylalkyl, (ggf. subst.);
A gleich CHOH, CO, CH=CH, C≡C, $CH_2$, O, S;
m gleich 1 oder 2;
n gleich 1 bis 3;
p gleich 0 bis 4;
R(5) gleich einigen Aminen
sowie ihre Salze weisen hervorragene calciumantagonistische Wirksamkeit auf.
Verfahren zu ihrer Herstellung werden beschrieben.

EP 0 303 180 A1

Xerox Copy Centre

## Benzothiazinon-oxide, Verfahren zu ihrer Herstellung, sie enthaltende Arzneimittel und deren Verwendung sowie Zwischenprodukte zu ihrer Herstellung

Es ist bekannt, daß Verbindungen, die das Einströmen von Calcium-Ionen in Zellen behindern, als Therapeutika zur Behandlung von verschiedenen Krankheiten, insbesondere des Herz-Kreislauf-Systems beim Menschen und anderen Warmblütern eingesetzt werden können.

Benzothiazinon-Derivate mit calciumantagonistischer Wirkung sind der deutschen Offenlegungsschrift 33 47 173 beschrieben, sowie in den deutschen Patentanmeldungen P 36 14 355.3, P 36 14 663.4, P 37 24 366.7 vorgeschlagen worden. Überraschenderweise wurde nun gefunden, daß die 1-Oxide bzw. die 1,1-Dioxide der in den genannten Patentanmeldungen beschriebenen bzw. vorgeschlagenen Verbindungen eine z.T. überlegene calciumantagonistische und/oder Herzkreislauf-Wirkung aufweisen.

Die Erfindung ist daher auf Benzothiazinon-Oxide der Formel I

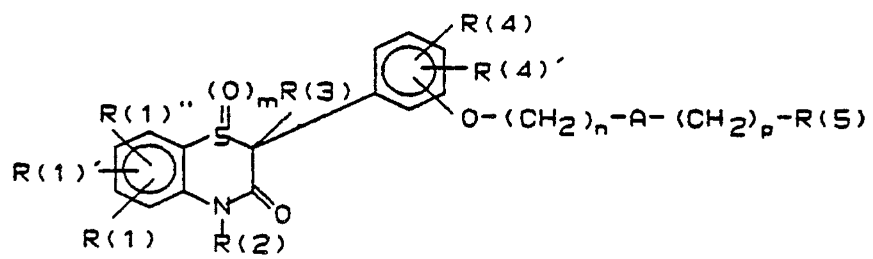

(I)

gerichtet, die calciumantagonistische Wirkung aufweisen, sowie deren Salze mit pharmazeutisch akzeptablen Säuren, in welcher Formel I bedeuten:

R(1), R(1)′ und R(1)″, gleich oder verschieden und voneinander unabhängig, Wasserstoff, ($C_1$-$C_4$)-Alkyl, ($C_1$-$C_3$)-Alkoxy, F, Cl, Br, $CF_3$, Nitro, Hydroxy, Acetamido oder Amino,

R(2) Wasserstoff, ($C_1$-$C_{10}$)-Alkyl, geradkettig oder verzweigt, ($C_3$-$C_{10}$)-Alkenyl, geradkettig oder verzweigt, Phenyl-($C_1$-$C_4$)-alkyl, wobei der Phenylring unsubstituiert oder durch einen, zwei oder drei Substituenten aus der Gruppe ($C_1$-$C_4$)-Alkyl, ($C_1$-$C_3$)-Alkoxy, F, Cl, $CF_3$, ($C_1$-$C_2$)-Alkylendioxy oder Nitro substituiert ist,

R(3) Wasserstoff, ($C_1$-$C_{15}$)-Alkyl, geradkettig oder verzweigt, ($C_3$-$C_{15}$)-Alkenyl, geradkettig oder verzweigt, ($C_4$-$C_8$)-Cycloalkyl, ($C_4$-$C_8$)-Cycloalkyl-($C_1$-$C_4$)-alkyl, Phenyl oder Phenyl-($C_1$-$C_4$)-alkyl, wobei der Phenylrest unsubstituiert oder durch einen, zwei oder drei Substituenten der Gruppe ($C_1$-$C_4$)-Alkyl, ($C_1$-$C_3$)-Alkoxy, F, Cl, $CF_3$, ($C_1$-$C_2$)-Alkylendioxy oder Nitro substituiert ist,

R(4) und R(4)′, gleich oder verschieden und voneinander unabhängig, Wasserstoff, ($C_1$-$C_4$)-Alkyl, ($C_1$-$C_3$)-Alkoxy, F, Cl, $CF_3$, Nitro, Hydroxy, Acetamido oder Amino,

A eine CH(OH)-Gruppe, eine C=O-Gruppe, eine CH=CH-Gruppe, eine C≡C-Gruppe, eine $CH_2$-Gruppe, Sauerstoff oder Schwefel,

m 1 oder 2,

n 1, 2 oder 3

p Null, 1, 2, 3 oder 4; sofern A ein Heteroatom ist, jedoch nur 2, 3 oder 4; und sofern A eine CH(OH), CH=CH- oder C≡C-Gruppe ist, jedoch nur 1, 2, 3 oder 4,

R(5) eine der folgenden Gruppen

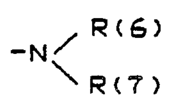

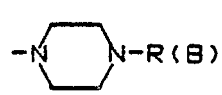

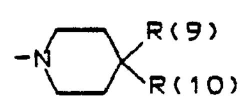

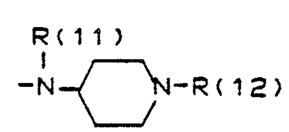

oder

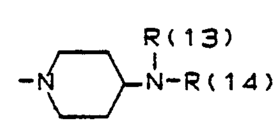

,

worin

R(6) und R(7),

gleich oder verschieden und voneinander unabhängig, Wasserstoff, ($C_1$-$C_{10}$)-Alkyl, ($C_4$-$C_8$)-Cycloalkyl, ($C_4$-$C_8$)-Cycloalkyl-($C_1$-$C_4$)-alkyl, Pyridyl-($C_1$-$C_4$)-alkyl, Phenyl-($C_1$-$C_6$)-alkyl, Benzhydryl oder Benzhydryl-($C_1$-$C_4$)-alkyl, wobei die Phenylreste jeweils unsubstituiert oder durch einen, zwei oder drei Reste aus der Gruppe ($C_1$-$C_4$)-Alkyl, ($C_1$-$C_4$)-Alkoxy, ($C_1$-$C_2$)-Alkylendioxy, F, Cl, Br, $CF_3$ oder Hydroxy substituiert sind,

R(8)

Wasserstoff, ($C_1$-$C_{10}$)-Alkyl, geradkettig oder verzweigt, ($C_1$-$C_8$)-Alkanoyl, Pyridyl, Pyrimidinyl, Phenyl, Phenyl-($C_1$-$C_4$)-alkyl, Phenyl-($C_3$-$C_5$)-alkenyl, Benzhydryl oder Benzhydryl-($C_1$-$C_4$)-alkyl, Phenyl-($C_1$-$C_4$)-alkanoyl oder Benzoyl, wobei die Phenylreste jeweils unsubstituiert oder durch eine, zwei oder drei Reste aus der Gruppe ($C_1$-$C_4$)-Alkyl, ($C_1$-$C_4$)-Alkoxy, ($C_1$-$C_2$)-Alkylendioxy, F, Cl, Br, $CF_3$ oder Hydroxy substituiert sind,

R(9)

Wasserstoff, ($C_1$-$C_{10}$)-Alkyl, Phenyl, Phenyl-($C_1$-$C_4$)-alkyl, wobei der Phenylrest jeweils unsubstituiert oder durch einen, zwei oder drei Reste aus der Gruppe ($C_1$-$C_4$)-Alkyl, ($C_1$-$C_4$)-Alkoxy, ($C_1$-$C_2$)-Alkylendioxy, F, Cl, Br, $CF_3$ oder Hydroxy substituiert ist,

R(10)

Wasserstoff, Hydroxy, oder ($C_1$-$C_4$)-Alkoxy, und

R(11) und R(12) bzw. R(13) und R(14),

gleich oder verschieden und voneinander unabhängig, Wasserstoff, ($C_1$-$C_{10}$)-Alkyl, geradkettig oder verzweigt, ($C_1$-$C_6$)-Alkanoyl, Phenyl-($C_1$-$C_4$)-alkyl, Benzylhydryl oder Benzhydryl-($C_1$-$C_4$)-alkyl, Phenyl-($C_1$-$C_4$)-alkanoyl, oder Benzoyl, wobei die Phenylreste jeweils unsubstituiert oder durch einen, zwei oder drei Reste aus der Gruppe ($C_1$-$C_4$)-Alkyl, ($C_1$-$C_4$)-Alkoxy, ($C_1$-$C_2$)-Alkylendioxy, F, Cl, Br, $CF_3$ oder Hydroxy substituiert sind, bedeuten,

sowie die Salze der Verbindungen der Formel I mit pharmazeutisch akzeptablen Säuren.

Bevorzugt werden die Verbindungen der Formel I, in welcher mindestens einer der Substituenten oder Indices folgende Bedeutung hat:

R(1) und R(1)′, gleich oder verschieden und voneinander unabhängig, Wasserstoff, Methyl, Ethyl, Methoxy, Ethoxy, Fluor, Chlor, $CF_3$, Nitro oder Acetamido,

R(1)″ Wasserstoff,

R(2) Wasserstoff, ($C_1$-$C_6$)-Alkyl, geradkettig oder verzweigt, Allyl, Methallyl, Benzyl, Phenethyl, 4-Methoxybenzyl, 3,4-Dimethoxybenzyl, 3,4,5-Trimethoxybenzyl, 3,4-Methylendioxybenzyl,

R(3) Wasserstoff, ($C_1$-$C_{12}$)-Alkyl, geradkettig oder verzweigt, Allyl, Methallyl, ($C_5$-$C_7$)-Cycloalkyl, ($C_5$-$C_7$)-Cycloalkyl-($C_1$-$C_4$)-alkyl, Benzyl, Methylbenzyl, Fluorbenzyl, Methoxybenzyl, Dimethoxybenzyl, Phenylethyl,

R(4) Wasserstoff, Methyl, Methoxy, Ethoxy, Fluor, Chlor, Nitro, Hydroxy, Acetamido oder Amino,

R(4)′ Wasserstoff,

A eine CH(OH)-Gruppe, eine C = O-Gruppe, eine CH = CH-Gruppe, eine C≡C-Gruppe, eine $CH_2$-Gruppe, Sauerstoff oder Schwefel,

m 1 oder 2,

n 1 oder 2,

p Null, 1, 2, 3; jedoch, wenn A ein Heteroatom ist, nur 2 oder 3; und, sofern A eine CH(CH), CH = CH oder C≡C-Gruppe ist, nur 1, 2 oder 3,

R(5) eine der folgenden Gruppen

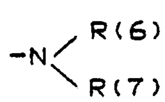
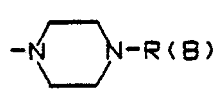
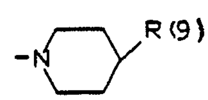

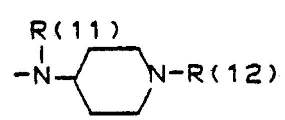
oder
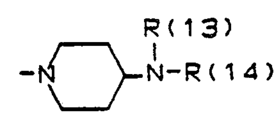

worin

R(6)

Wasserstoff, Methyl, Ethyl, Propyl, Isopropyl,

R(7)
Wasserstoff, Methyl, Ethyl, Propyl, Isopropyl, Cyclopentylethyl, Cyclohexylethyl, Phenyl-($C_1$-$C_4$)- alkyl, Benzhydryl oder Benzhydryl-($C_1$-$C_4$)-alkyl, wobei die Phenylreste jeweils unsubstituiert oder durch einen, zwei oder drei Reste aus der Gruppe ($C_1$-$C_4$)-Alkyl, ($C_1$-$C_4$)-Alkoxy, ($C_1$-$C_2$)-Alkylendioxy, F, Cl, $CF_3$ oder Hydroxy substituiert sind, Pyridyl-($C_1$-$C_4$)-alkyl,
R(8)
Wasserstoff, ($C_1$-$C_6$)-Alkyl, geradkettig oder verzweigt, ($C_1$-$C_6$)-Alkanoyl, Phenyl, wobei der Phenylrest durch einen oder zwei Reste aus der Gruppe ($C_1$-$C_4$)-Alkyl, ($C_1$-$C_4$)-Alkoxy, ($C_1$-$C_2$)-Alkylendioxy, F, Cl, $CF_3$ oder Hydroxy substituiert sein kann, Phenyl-($C_1$-$C_4$)-alkyl, Phenyl-($C_3$-$C_5$)-alkenyl, Benzhydryl oder Benzhydryl-($C_1$-$C_4$)-alkyl, Phenyl-($C_1$-$C_4$)-alkanoyl oder Benzoyl, wobei die Reste jeweils substituiert oder durch einen, zwei oder drei Reste aus der Gruppe Methyl, Ethyl, Methoxy, Ethoxy, ($C_1$-$C_2$)-Alkylendioxy, F, Cl $CF_3$ oder Hydroxy substituiert sind,
R(9)
Phenyl, Phenyl-($C_1$-$C_4$)-alkyl, wobei der Phenylrest jeweils unsubstituiert oder durch einen, zwei oder drei Reste aus der Gruppe ($C_1$-$C_4$)-Alkyl, ($C_1$-$C_4$)-Alkoxy, ($C_1$-$C_2$)-Alkylendioxy, F, Cl, $CF_3$ oder Hydroxy substituiert ist,
R(10)
Wasserstoff, Hydroxy oder Methoxy
R(11), R(12), R(13) und R(14),
gleich oder verschieden, Wasserstoff, ($C_1$-$C_8$)-Alkyl, ($C_1$-$C_6$)-Alkanoyl, Phenyl-($C_1$-$C_4$)-alkyl, Benzhydryl oder Benzhydryl-($C_1$-$C_4$)-alkyl, Phenyl-($C_1$-$C_4$)-alkanoyl oder Benzoyl, wobei die Phenylreste jeweils unsubstituiert oder durch einen, zwei oder drei Reste aus der Gruppe Methyl, Ethyl, Methoxy, Ethoxy, ($C_1$-$C_2$)-Alkylendioxy, F, Cl, $CF_3$ oder Hydroxy substituiert sind, bedeuten
sowie die Salze dieser Verbindungen der Formel I mit pharmazeutisch akzeptablen Säuren.

Besonders bevorzugt werden Verbindungen der Formel I, in welchen mindestens einer der Substituenten oder Indices bedeutet:

R(1) Wasserstoff, Methyl, Methoxy, Fluor oder Chlor,
R(1)' Wasserstoff oder Methoxy,
R(1)'' Wasserstoff,
R(2) Wasserstoff, Methyl, Ethyl, Propyl, Isopropyl, Butyl, sec. Butyl, Isobutyl, Benzyl, Phenethyl,
R(3) Wasserstoff, ($C_1$-$C_{12}$)-Alkyl, geradkettig oder verzweigt, Cyclopentyl, Cyclohexyl, Cyclopentylmethyl, Cyclohexylmethyl, Allyl, Methallyl, Benzyl, Methylbenzyl, Fluorbenzyl, Methoxybenzyl, Dimethoxybenzyl, Phenylethyl,
R(4) Wasserstoff, Methoxy, Methyl, Fluor, Chlor, Nitro oder Hydroxy,
R(4)' Wasserstoff,
A eine CH(OH)-Gruppe, eine C=O-Gruppe, eine CH=CH-Gruppe, eine C≡C-Gruppe, eine $CH_2$-Gruppe oder Sauerstoff,
m 1 oder 2,
n 1 oder 2,
p Null, 1 oder 2; jedoch, wenn A ein Heteroatom ist, nur 2; und sofern A eine CH(OH), CH=CH oder C≡C-Gruppe ist, nur 1 oder 2,
R(5) eine der folgenden Gruppen

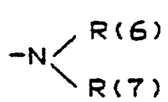 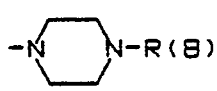 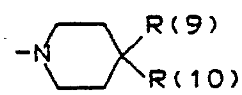

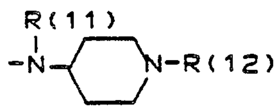 oder 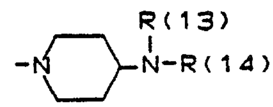 ,

worin
R(6)
Wasserstoff oder Methyl,
R(7)
Phenyl-($C_1$-$C_4$)-alkyl, Benzhydryl oder Benzhydryl-($C_1$-$C_4$)-alkyl, wobei der Phenylrest jeweils unsubstituiert

oder durch einen, zwei oder drei Reste aus der Gruppe Methyl, Methoxy, Fluor, Chlor, Methylendioxy oder Hydroxy substituiert ist,

R(8)

$(C_1-C_6)$-Alkyl, geradkettig oder verzweigt, $(C_1-C_6)$-Alkanoyl, Phenyl, Phenyl-$(C_1-C_4)$-alkyl, Benzhydryl oder Benzhydryl-$(C_1-C_4)$-alkyl, Phenyl-$(C_1-C_4)$-alkanoyl oder Benzoyl, wobei die Phenylreste jeweils unsubstituiert oder durch einen, zwei oder drei Reste aus der Gruppe Methyl, Methoxy, Ethoxy, Methylendioxy, Fluor, Chlor oder Hydroxy substituiert sind,

R(9)

Phenyl, wobei der Phenylrest unsubstituiert oder durch einen, zwei oder drei Reste aus der Gruppe Methyl, Methoxy, Fluor, Chlor, Methylendioxy oder Hydroxy substituiert ist,

R(10)

Wasserstoff, Hydroxy oder Methoxy,

R(11), R(12), R(13) und R(14),

gleich oder verschieden sind, $(C_1-C_6)$-Alkyl, $(C_1-C_6)$-Alkanoyl, Phenyl-$(C_1-C_4)$-alkyl, Benzhydryl oder Benzhydryl-$(C_1-C_4)$-alkyl, Phenyl-$(C_1-C_4)$-alkanoyl oder Benzoyl, wobei die Phenylreste jeweils unsubstituiert oder durch einen, zwei oder drei Reste aus der Gruppe Methyl, Methoxy, Methylendioxy, Fluor, Chlor oder Hydroxy substituiert sind, bedeuten

sowie die Salze dieser Verbindungen der Formel I mit pharmazeutisch akzeptablen Säuren.

Als solche pharmazeutisch akzeptablen Säuren kommen anorganische Säuren wie Salzsäure, Bromwasserstoffsäure, Jodwasserstoffsäure, Schwefelsäure, Phosphorsäure oder Salpetersäure oder organische Säuren wie Weinsäure, Äpfelsäure, Milchsäure, Maleinsäure, Fumarsäure, Malonsäure, Oxalsäure, Gluconsäure, Camphersulfonsäure, Benzolsulfonsäure, Essigsäure, Propionsäure oder p-Toluolsulfonsäure in Betracht.

Die Verbindungen der Formel I weisen asymmetrische C-Atome auf und können daher als Enantiomere oder Diastereomere auftreten. Die Erfindung umfaßt sowohl die reinen Isomeren als auch deren Gemische. Diese Gemische von Diastereomeren können nach gebräuchlichen Methoden, zum Beispiel selektive Kristallisation aus geeigneten Lösungsmittel oder Chromatographie an Kieselgel oder Aluminiumoxid in die Komponenten aufgetrennt werden. Racemate können nach üblichen Methoden in die einzelnen Enantiomeren aufgetrennt werden, so zum Beispiel durch Salzbildung mit optisch aktiven Säuren wie Camphersulfonsäure oder Dibenzoylweinsäure und selektive Kristallisation, oder durch Derivatisierung mit geeigneten optisch aktiven Reagenzien, Trennung der diastereomeren Derivate und Rückspaltung.

Die Erfindung betrifft weiterhin Verfahren zur Herstellung von Verbindungen der Formel I, dadurch gekennzeichnet, daß man

a) eine Verbindung der Formel II

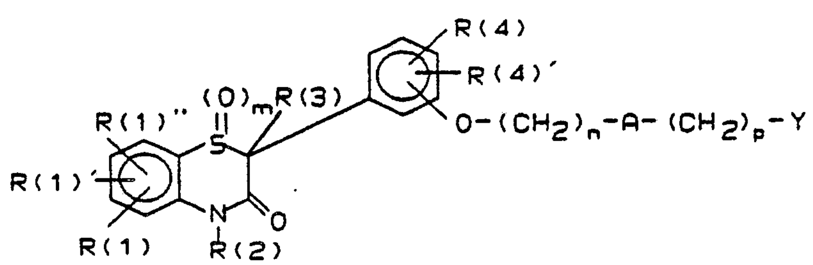

(II),

in welcher R(1), R(1)′, R(1)″, R(2), R(3), R(4), R(4)′, A, m, n und p die gleiche Bedeutung wie in Formel I haben und in welcher Y eine Abgangsgruppe, die nucleophil verdrängt werden kann, insbesondere ein Chlor-, Brom- oder Jodatom, einen Sulfonsäurerest, vorzugsweise einen Methansulfonyloxyrest, einen Benzolsulfonyloxyrest, einen Toluolsulfonyloxyrest oder einen Trifluormethansulfonyloxyrest bedeutet, mit einer der Verbindungen der Formel IIIa, IIIb, IIIc, IIId oder IIIe

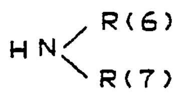 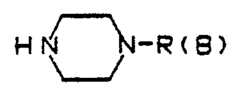 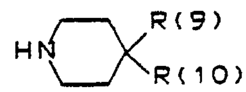

IIIa IIIb IIIc

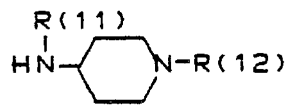

oder

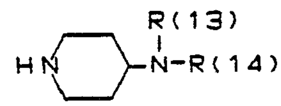

IIId IIIe

in welchen R(6), R(7), R(8), R(9), R(10), R(11), R(12), R(13) und R(14) die gleiche Bedeutung wie in Formel I haben, unter Bedingungen einer nucleophilen Substitution, vorzugsweise in einem polaren organischen Lösungsmittel wie einem Alkohol, vorzugsweise Methanol, Ethanol, Propanol oder Isopropanol oder einem niederen Keton, vorzugsweise Aceton oder Methylethylketon oder Dimethylformamid, Dimethylsulfoxid oder Sulfolan oder einem Kohlenwasserstoff, vorzugsweise Toluol, mit oder ohne Gegenwart einer Hilfsbase zum Abfangen der sich bildenden Säure, vorzugsweise in Gegenwart von Kaliumcarbonat, Natriumcarbonat, Triethylamin, N-Ethylmorpholin oder Pyridin, bei einer Temperatur zwischen 0 und 160° C, vorzugsweise zwischen 20 und 120° C, umsetzt, oder daß man

b) eine Verbindung der Formel IV,

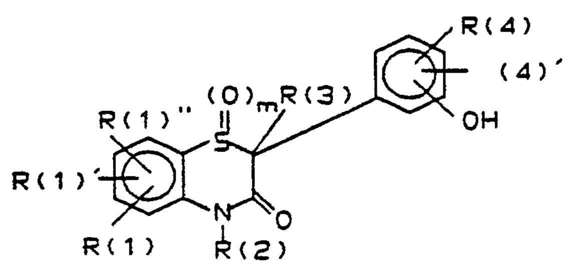

(IV) ,

in welcher R(1), R(1)′, (R(1)″, R(2), R(3), R(4), R(4)′ und m die gleiche Bedeutung wie in Formel I haben, mit einer Verbindung der Formel V,

$Z-(CH_2)_n-A-(CH_2)_p-R(5)$ (V)

in welcher Z gleich wie Y in Formel II definiert ist und in welcher R(5), A, n und p die gleiche Bedeutung wie in Formel I haben, entweder in einem polaren aprotischen Lösungsmittel wie Dimethylformamid, Dimethylsulfoxid, Tetrahydrofuran, Sulfolan oder N-Methylpyrrolidon, in Gegenwart einer starken Base wie Natriumhydrid, Kaliumhydrid, Natriumamid, Lithiumdiisopropylamid, Butyllithium oder Lithiumhexamethyldisilazid, bei einer Temperatur zwischen -40 und +60° C, vorzugsweise zwischen -10 und -30° C, oder in einem protischen oder aprotischen polaren organischen Lösungsmittel wie einem niederen Alkohol, beispielsweise Methanol, Ethanol, Isopropanol oder einem niederen Keton, vorzugsweise Aceton oder Methylethylketon oder in Dimethylformamid, in Gegenwart einer schwachen bis mittelstarken Base wie einem Alkali- oder Erdalkalimetallhydroxid oder -carbonat oder einem Amin wie beispielsweise Triethylamin, N-Ethylmorpholin, N-Methyldiisopropylamin oder Pyridin, bei einer Temperatur zwischen 0 und 160° C, vorzugsweise zwischen 20 und 120° C, umsetzt, oder daß man

c) eine Verbindung der Formel VI

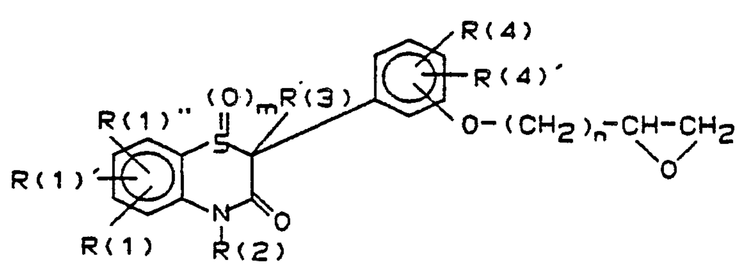

(VI),

in welcher R(1), R(1)′, R(1)″, R(2), R(3), R(4), R(4)′, m und n die gleiche Bedeutung wie in Formel I haben, mit Aminen der Formel IIIa - IIIe ohne Lösungsmittel oder in Gegenwart eines vorzugsweisae polaren Lösungsmittels wie Methanol, Isopropanol, Aceton, THF oder Dimethylformamid umsetzt, wobei Verbindungen der Formel I entstehen, worin A = CH(OH) und p = 1 bedeutet oder daß man

d) eine Verbindung der Formel VII

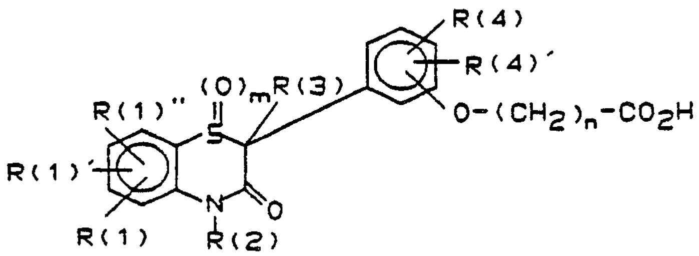

(VII),

in welcher R(1), R(1)′, R(1)″, R(2), R(3), R(4), R(4)′, m und n die gleiche Bedeutung wie in Formel I haben, mit einer der Verbindungen der Formeln IIIa, IIIb, IIIc, IIId oder IIIe unter aus der Literatur bekannten Amidbildungsbedingungen umsetzt, wobei Verbindungen der Formel I entstehen, in denen A eine C=O-Gruppe und p = Null bedeutet,

oder daß man

e) eine Verbindung der Formel VIII

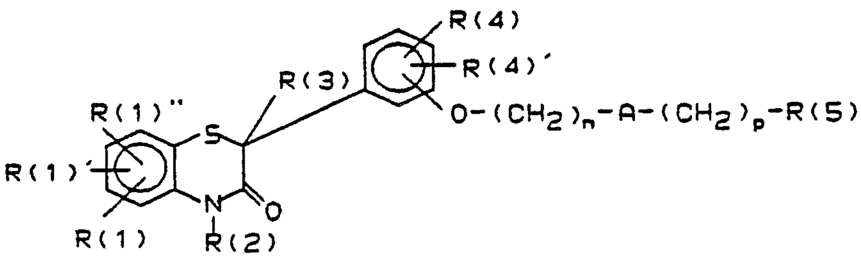

(VIII),

in welcher R(1), R(1)′, R(1)″, R(2), R(3), R(4), R(4)′, R(5), A, n und p die gleiche Bedeutung wie in Formel I haben, mit Oxydationsmitteln, die bekanntermaßen Thioether in Sulfoxide bzw. Sulfone überführen, wie z.B. m-Chlorperbenzoesäure, Peressigsäure, Wasserstoffperoxid, Kaliumpermanganat oder Chromsäure, zu Verbindungen der Formel I oxidiert.

Verbindungen der Formel VIII sind z.B. in der deutschen Offenlegungsschrift 33 47 173 beschrieben, sowie in den deutschen Patentanmeldungen P 36 14 355.3, P 35 14 363.4 und P 37 24 366.7 vorgeschlagen worden.

Verbindungen der Formel II erhält man aus Verbindungen der Formel IX

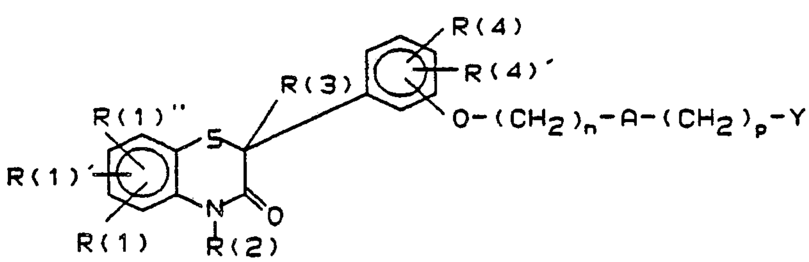

(IX),

in welcher R(1), R(1)′, R(1)″, R(2), R(3), R(4), R(4)′, A, m, n und p die gleiche Bedeutung wie in Formel I haben und in welcher Y dieselbe Bedeutung wie in Formel II hat, mit Oxydationsmitteln unter den zu e) beschriebenen Bedingungen. Verbindungen der Formel IX sind in der obengenannten deutschen Offenlegungsschrift DOS 33 47 173 bzw. P 36 14 355.3, P 36 14 363.4, P 37 24 366.7 beschrieben bzw. vorgeschlagen worden.

Verbindungen der Formel II können auch hergestellt werden aus Verbindungen der Formel IV und Verbindungen der Formel X

$Z-(CH_2)_n-A-(CH_2)_p-Y$ (X),
worin A, n und p die gleiche Bedeutung wie in Formel I haben, Y die gleiche Bedeutung wie in Formel II und Z die gleiche Bedeutung wie in Formel V haben.

Verbindungen der Formel IV enthält man aus Verbindungen der Formel XI

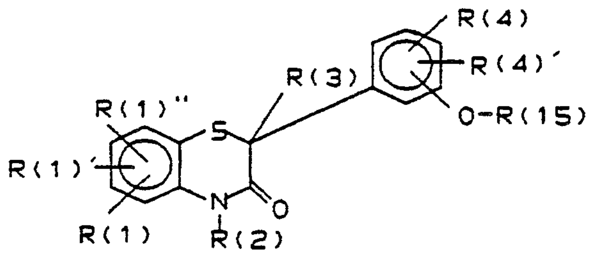

(XI),

in welcher R(1), R(1)′, R(1)″, R(2), R(3), R(4) und R(4)′ die gleiche Bedeutung wie in Formel I haben und R-(15) Wasserstoff oder eine unter milden Bedingungen abspaltbare Schutzgruppe, beispielsweise eine Methyl-, Benzyl- oder Acetylgruppe, darstellt, mit Oxydationsmitteln unter den zu e) beschriebenen Bedingungen und ggf. anschließende Abspaltung der Schutzgruppe R(15) unter geeigneten Bedingungen, beispielsweise durch katalytische Hydrierung für die Benzylgruppe, Umsetzung mit Bortribromid, Bortrichlorid, Trimethyljodsilan oder Pyridinhydrochlorid für die Methylgruppe oder Kaliumcarbonat in alkoholischer Lösung für die Acetylgruppe. Die Herstellung der Verbindungen der Formel XI ist z.B. in der deutschen Offenlegungsschrift 33 47 173 beschrieben.

Verbindungen der Formel VI erhält man aus Verbindungen der Formel IV durch Alkylierung mit Verbindungen der Formel XII

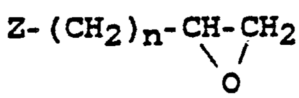

(XII),

worin Z die gleiche Bedeutung wie in Formel V hat, unter den für Verfahren b) beschriebenen Bedingungen.

Verbindungen der Formel VI lassen sich auch herstellen aus Verbindungen der Formel XIII

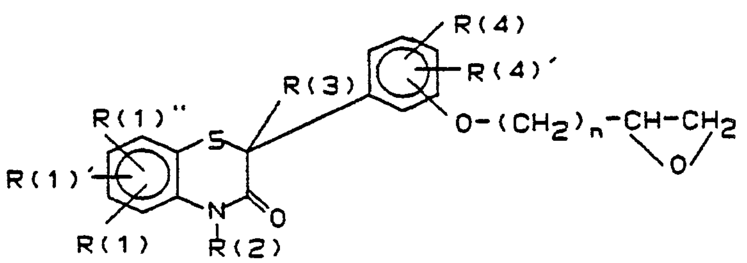

(XIII),

in welcher R(1), R(1)′, R(1)″, R(2), R(3), R(4) und R(4)′ die gleiche Bedeutung wie in Formel I haben, durch Oxydation mit Oxydationsmitteln unter den zu e) beschriebenen Bedingungen. Die Herstellung von Verbindungen der Formel XIII ist z.B. in der deutschen Patentanmeldung P 36 14 355.3 vorgeschlagen:

Eine Verbindung der Formel XIII

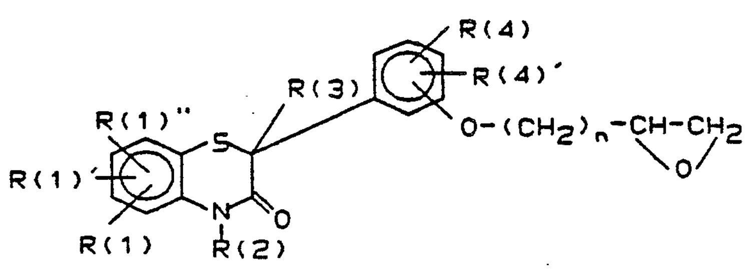

(XIII)

erhält man aus Verbindungen der Formel XI

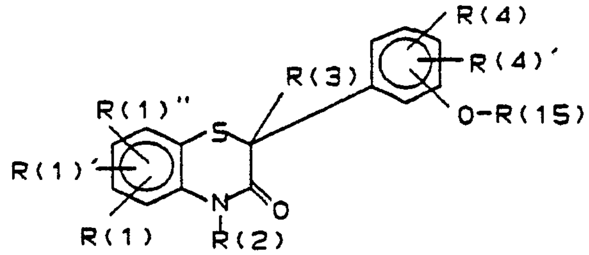

(XI),

mit R(15) gleich Wasserstoff)
z.B. mit Epichlorhydrin und Basen (für n = 1) nach bekannten Methoden oder durch Alkylierung von Verbindungen der Formel mit Verbindungen der Formel X′
$Z-(CH_2)-CH=CH_2$ X′,
worin n die gleiche Bedeutung hat wie in Formel I und worin Z die gleiche Bedeutung wie in Formel X hat, wobei Verbindungen der Formel XIV

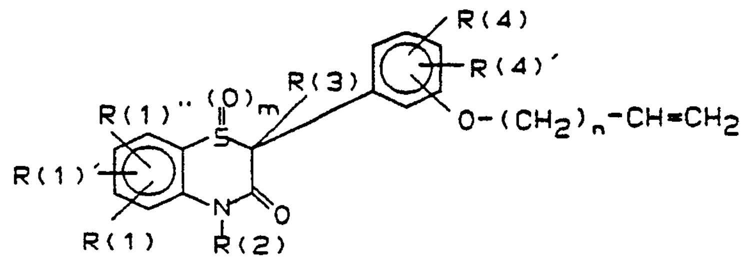

XIV

(mit m = Null)
gebildet werden. Anschließende Epoxidation der Verbindungen nach bekannten Verfahren, beispielsweise mit m-Chlorperbenzoesäure in Methylenchlorid ergibt Verbindungen der Formel XIII.

Verbindungen der Formel VI lassen sich auch herstellen aus Verbindungen der Formel XIV

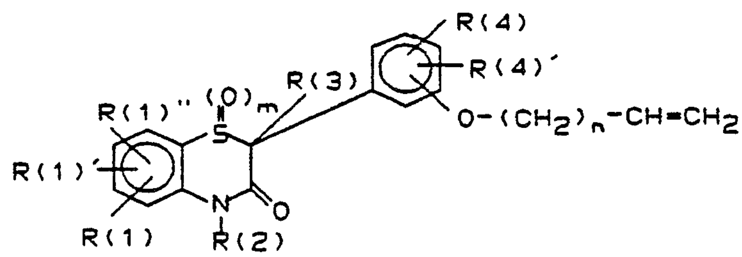

(XIV),

worin R(1), R(1)′, R(1)″, R(2), R(3), R(4), R(4)′, m und n die gleiche Bedeutung wie in Formel I haben, durch Epoxydation nach bekannten Verfahren, beispielsweise mit m-Chlorbenzoesäure oder Peressigsäure in Methylenchlorid.

Verbindungen der Formel XIV erhält man aus Verbindungen der Formel IV durch Alkylierung mit Verbindungen der Formel XV
$Z-(CH_2)_n-CH=CH_2$ (XV)
worin n die gleiche Bedeutung wie in Formel I und Z die gleiche Bedeutung wie in Formel V hat.

Verbindungen der Formel VII lassen sich herstellen aus Verbindungen der Formel IV durch Alkylierung mit Verbindungen der Formel XVI
$Z-(CH_2)_n-CO_2R(16)$ (XVI),
worin Z die gleiche Bedeutung wie in Formel V hat und R(16) Wasserstoff oder einen Alkylrest bedeutet unter den für Verfahren b) beschriebenen Bedingungen,
oder durch Oxydation von Verbindungen der Formel XVII

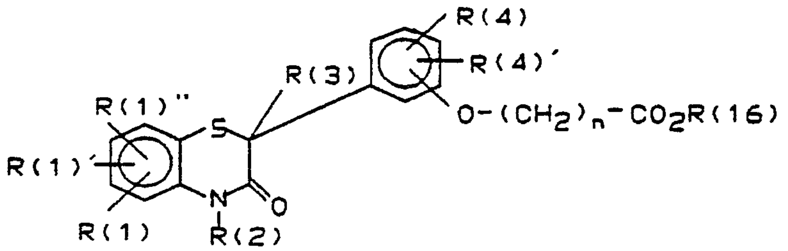

(XVII),

worin R(1), R(1)′, R(1)″, R(2), R(3), R(4), R(4)′ und n die gleiche Bedeutung wie in Formel I haben und R(16) die gleiche Bedeutung wie in Formel XVI mit Oxydationsmitteln wie unter Verfahren e) beschrieben.

Alkyl, Alkylen, Alkanoyl, Alkoxy bedeuten, wenn nicht ausdrücklich anders erwähnt, immer gerade oder verzweigte Ketten.

Der erfindungsgemäßen Verbindungen der Formel I weisen pharmakologische und biochemische Wirkungen, insbesondere calciumantagonistische und blutdrucksenkende Wirkungen auf und können daher zur Behandlung aller Krankheitszustände, die auf einer Störung in dem Calciumhaushalt eines Warmblüters beruhen, verwendet werden.

Ihre calciumantagonistische Wirksamkeit kann an dem biochemischen Testmodell der Verdrängung von tritiummarkiertem Nitrendipin gezeigt werden.

Hierbei werden Membranpräparationen, die isolierte Calciumkänale enthalten, mit der markierten Substanz beladen. Nach Inkubation mit der Testsubstanz wird die freigesetzte Radioaktivität in der überstehenden Lösung bestimmt. In diesem Modell weisen die erfindungsgemäßen Verbindungen der Formel I $IC_{50}$-Werte von $10^{-5}$molar bis $10^{-10}$molar auf.

In weiteren Testmodellen, mit denen calciumantagonistische Wirkung nachgewiesen werden kann, z.B. an der Coronardurchströmung am isolierten Meerschweinchenherzen oder am Aktionspotential des isolierten Meerschweinchenpapillarmuskels sind Verbindungen der Formel I ebenfalls stark wirksam.

Die erfindungsgemäßen Verbindungen der Formel I und ihre pharmakologisch verträglichen Salze vermindern das Einströmen von Calcium-Ionen in Zellen und eignen sich daher zur Behandlung des Herz-Kreislaufsystems bei entsprechenden Beschwerden, z.B. bei verschiedenen Formen der Angina pectoris, Tachycardie, Herzrhythmusstörungen und Bluthochdruck. Sie sind innerhalb eines breiten Dosisbereichs wirksam. Die Höhe der verabreichten Dosis ist abhängig von der Art der gewünschten Behandlung, von der Verabreichungsweise, vom Zustand, vom Typ und von der Größe des behandelten Säugers. Bei oraler Dosierung werden befriedrigende Ergebnisse mit Dosen von ab 0,01, vorzugsweise ab 0,1, insbesondere ab 0,5 mg und bis zu 100 mg, vorzugsweise bis zu 20 mg, insbesondere bis zu 15 mg einer Verbindung der Formel I pro kg Körpergewicht erreicht. Beim Menschen variiert die tägliche Dosis von mindestens 10, insbesondere 20 mg, bis höchstens 800 mg, vorzugsweise 500 mg, wobei Einzeldosen von 5 bis 200 mg, insbesondere 5 bis 100 mg, vorzugsweise ein- bis dreimal täglich, gegeben werden können. Diese Angaben beziehen sich auf einen Erwachsenen vom Körpergewicht von ca. 75 kg.

Für intravenöse und intramuskuläre Anwendung beträgt die Dosis mindestens 1 mg, vorzugsweise mindestens 5 und höchstens 300 mg, vorzugsweise bis zu 150 mg täglich.

Die pharmakologisch verwendbaren Verbindungen der vorliegenden Erfindung und ihre Salze können zur Herstellung von pharmazeutischen Präparaten verwendet werden, welche eine wirksame Menge der Aktivsubstanz zusammen mit Trägerstoffen enthalten und die sich zur enteralen und parenteralen Verabreichung eignen. Vorzugsweise verwendet werden Tabletten und Gelatinekapseln, welche den Wirkstoff zusammen mit Verdünnungsmitteln, z.B. Lactose, Dextrose, Rohrzucker, Mannitol, Sorbitol, Cellulose und/oder Glycin und Gleitmitteln wie Kieselerde, Talk, Stearinsäure oder deren Salze, wie Magnesium- oder Calciumstearat, und/oder Polyethylenglykol enthalten. Tabletten enthalten ebenfalls Bindemittel wie Magnesiumaluminiumsilicat, Stärke, Gelatine, Traganth, Methylcellulose, Natriumcarboxymethylcellulose und/oder Polyvinylpyrrolidon und, falls benötigt, Farbstoff, Geschmacksstoffe und Süßmittel. Injizierbare Lösungen sind vorzugsweise isotonische wäßrige Lösungen oder Suspensionen, die sterilisiert sein können und Hilfsmittel wie Konservier-, Stabilisierungs-, Netz- und/oder Emulgiermittel, Löslichkeitsvermittler, Salze zur Regulierung des osmotischen Drucks und/oder Puffersubstanzen enthalten können. Die erfindungsgemäßen pharmazeutischen Präparate, die, wenn gewünscht, weitere pharmakologisch wertvolle Stoffe enthalten können, werden z.B. mittels konventioneller Misch-, Granulier- und Dragierverfahren, hergestellt und enthalten 0,1 % bis etwa 75 %, bevorzugt etwa 1 % bis etwa 50 % des Wirkstoffs.

Die im Anschluß folgenden Beispiele sollen die Erfindung erläutern, ohne sie auf diese Beispiele zu begrenzen.

**Beispiel 1**

2-[2-[4-[4-[2-(3,4,5-Trimethoxyphenyl)-ethyl]-piperazin-1-yl]-butoxy]-phenyl]-2-isopropyl-4-methyl-2,3-dihydrobenzothiazin-3-on-1,1-dioxid

a) 7,2 g 2-(2-[4-Brombutoxy]-phenyl)-2-isopropyl-4-methyl-2,3-dihydrobenzothiazin-3-on-1,1-dioxid werden in 30 ml DMF und 22,5 ml 2N NaOH mit 7,82 g 1-(2-[3,4,5-Trimethoxyphenyl]-ethyl)-piperazin-dihydrochlorid 4 Stunden auf 80 bis 90° C erwärmt. Die Reaktionsmischung wird auf 300 ml Eiswasser gegeben und mehrmals mit Ethylacetat extrahiert. Die vereinigten organischen Phasen werden mit Wasser gewaschen, getrocknet und im Vakuum eingeengt. Der Rückstand wird an 200 g Kieselgel chromatographiert (Elutionsmittel Dichlormethan/Methanol 9 : 1). Nach Eindampfen der Produktfraktionen wird aus Ethanol mit ethanolischer HCl das Dihydrochlorid gefällt und aus Ethanol umkristallisiert.
Fp. 223 - 224° C
$^1$H-NMR (Base) : $\delta$ = 6,45-8,0 (m,8H), 6,40 (s,2H), 3,80 (s,6H), 3,77 (s,3H), 3,45 (s,3H), 1,40-2,0 (m,4H), 1,25 (d,3H), 1,07 (d,3H)

b) Das als Ausgangsmaterial verwendete 2-(2-[Brombutoxy]-phenyl-2-isopropyl-4-methyl-2,3-dihydrobenzothiazin-3-on-1,1-dioxid läßt sich wie folgt herstellen:
8,9 g 2-(2-[4-Brombutoxy]-phenyl)-2-isopropyl-4-methyl-2,3-dihydrobenzothiazin-3-on wird in 400 ml Dichlormethan gelöst und bei Zimmertemperatur portionsweise mit 17,2 g 3-Chlorperbenzoesäure versetzt. Man rührt 4 Stunden nach und wäscht dann das Reaktionsgemisch 5 mal mit gesättigter $NaHCO_3$-Lösung. Die organische Phase wird mit Natriumsulfat getrocknet und im Vakuum eingeengt. Das Produkt kristallisiert beim Stehen über Nacht.
Fp. 118 bis 120° C
$^1$H-NMR : $\delta$ = 6,5-8,0 (m,8H), 3,83 (m,2H), 3,45 (s,3H), 2,8-3,6 (m,3H), 1,6-2,2 (m,4H), 1,08 (dd,6H)

**Beispiel 2**

(+)-Enantiomeres von 2-[2-[4-[4-[2-(3,4,5-Trimethoxyphenyl)-ethyl]-piperazin-1-yl]-butoxy]-phenyl]-2-isopropyl-4-methyl-2,3-dihydrobenzothiazin-3-on-1,1-dioxid

Die Herstellung erfolgt analog dem Beispiel 1, wobei das (+)-Enantiomere von 2-(2-[4-Brombutoxy]-phenyl)-2-isopropyl-4-methyl-2,3-dihydrobenzothiazin-3-on-1,1-dioxid als Reaktionspartner verwendet wird. Das Dihydrochlorid schmilzt bei 243-4° C (Zers.)
$^1$H-NMR (Base) : $\delta$ = 6,5-7,9 (m,8H), 6,35 (s,2H), 3,84 (s,6H), 3,80 (s,3H), 3,43 (s,3H), 1,5-1,9 (m,4H), 1,27 (d,3H), 1,15 (dd,6H)

**Beispiel 3**

(-)-Enantiomeres von 2-[2-[4-[4-[2-(3,4,5-Trimethoxyphenyl)-ethyl]-piperazin-1-yl]-butoxy]-phenyl]-2-isopropyl-4-methyl-2,3-dihydrobenzothiazin-3-on-1,1-dioxid

Die Herstellung erfolgt analog wie bei der racemischen Verbindungen (Beispiel 1a) mit dem (-)-Enantiomeren von 2-(2-[4-Brombutoxy]-phenyl)-2-isopropyl-4-methyl-2,3-dihydrobenzothiazin-3-on-1,1-dioxid. Das Dihydrochlorid schmilzt bei 241-3° C (Zers.)
Das $^1$H-NMR-Spektrum ist mit dem des (+)-Enantiomeren (Beispiel 2) identisch.

**Beispiel 4**

2-[2-[4-[4-(3,4,5-Trimethoxyphenylacetyl)-piperazin-1-yl]-butoxy]-phenyl]-2-isopropyl-4-methyl-2,3-dihydrobenzothiazin-3-on-1,1-dioxid

Herstellung analog Beispiel 1a aus 2-(2-[4-Brombutoxy]-phenyl)-2-isopropyl-4-methyl-2,3-dihydrobenzothiazin-3-on-1,1-dioxid und 1-(2-[3,4,5-Trimethoxyphenyl]-acetyl)-piperazin. Hydrochlorid Fp. 145 °C.
$^1$H-NMR (Base) : $\delta$ = 6,53-8,05 (m,8H), 6,43 (s,2H), 3,83 (s,6H), 3,80 (s,3H), 3,65 (s,2H), 3,43 (m,3H), 1,70 (m,4H), 1,16 (dd, 6H)

**Beispiel 5**

(+)-Enantiomeres von 2-[2-[4-[4-[N-Methyl-2-(3,4,5-trimethoxyphenyl)-ethylamino]-piperidin-1-yl]-butoxy]-phenyl]-2-isopropyl-4-methyl-2,3-dihydrobenzothiazin-3-on-1,1-dioxid

Die Synthese erfolgt analog Beispiel 1a aus (+)-2-(2-[4-Brombutoxy]-phenyl)-2-isopropyl-4-methyl-2,3-dihydrobenzothiazin-3-on-1,1-dioxid und 4-(N-Methyl-2-[3,4,5-trimethoxyphenyl]-ethylamino)-piperazin.
$^1$H-NMR : $\delta$ = 6,5-8,0 (m,8H), 6,40 (s,2H), 3,80 (s,6H), 3,76 (s,3H), 3,43 (s,3H), 1,4-2,2 (m,8H), 0,9-1,4 (dd,6H)

**Beispiel 6**

2-[2-[4-[4-[2-(3,4,5-Trimethoxyphenyl)-ethyl]-piperazin-1-yl]-butoxy]-phenyl]-2-benzyl-4-methyl-2,3-dihydrobenzothiazin-3-on-1,1-dioxid

Die Synthese erfolgt analog Beispiel 1a aus 2-(2-[4-Brombutoxy]-phenyl)-2-benzyl-4-methyl-2,3-dihydrobenzothiazin-3-on-1,1-dioxid und 1-(2-[3,4,5-Trimethoxyphenyl]-ethyl)-piperazin.
Dihydrochlorid Fp. 201-3 °C (Zers.)
$^1$H-NMR (Base) $\delta$ = 6,45-7,8 (m,8H), 6,9 (s,5H), 6,38 (s,2H), 3,80 (s,6H), 3,77 (s,3H), 3,37 (s,3H), 3,2-4,2 (m,4H), 2,0-2,8 (m,14H), 1,2-1,8 (m,4H)
Das als Ausgangsmaterial benötigte 2-(2-[4-Brombutoxy]-phenyl)-2-benzyl-4-methyl-2,3-dihydrobenzothiazin-3-on-1,1-dioxid wird durch Oxidation von 2-(2-[4-Brombutoxy]-phenyl)-2-benzyl-4-methyl-2,3-dihydrobenzothiazin-3-on hergestellt analog Beispiel 1b.

**Beispiel 7**

2-[2-[4-[N-Methyl-2-(3,4-Dimethoxyphenyl)-ethylamino]-butoxy]-phenyl]-2-benzyl-4-methyl-2,3-dihydrobenzothiazin-3-on-1,1-dioxid

Die Herstellung erfolgt analog Beispiel 1a mit N-Methylhomoveratrylamin als Base.
Oxalat: Fp. 161-163 °C
$^1$H-NMR : $\delta$ = 6,5-7,8 (m,11H), 6,86 (s,5H), 3,8 (s,6H), 3,33 (s,3H), 3,3-4,2 (s,4H), 2,2-3,0 (m,6H), 2,30 (s,3H), 1,2-2,0 (m,4H)

**Beispiel 8**

2-[2-[4-[4-[2-(3,4,5-Trimethoxyphenyl)-ethyl]-piperazin-1-yl]-butoxy]-5-fluorphenyl]-2-isopropyl-4-methyl-2,3-dihydrobenzothiazin-3-on-1,1-dioxid

Die Herstellung erfolgt analog Beispiel 1a aus 2-(2-[4-Brombutoxy]-5-fluorphenyl)-2-isopropyl-4-methyl-2,3-dihydrobenzothiazin-3-on-1,1-dioxid.
Dihydrochlorid: Fp. 224-226 °C
$^1$H-NMR : δ = 6,6-8,2 (m,7H), 6,45 (s,2H), 3,85 (s,6H), 3,80 (s,3H), 3,50 (s,3H), 1,80 (s,4H), 1,20 (dd,6H)

## Beispiel 9

2-[2-[4-[4-[2-(3,4,5-Trimethoxyphenyl)-ethyl]-piperazin-1-yl]-but-2-in-oxy]-5-fluorphenyl]-2-isopropyl-4-methyl-2,3-dihydrobenzothiazin-3-on-1,1-dioxid

a) Die Herstellung erfolgt analog Beispiel 1a aus 2-(2-[4-Chlorbut-2-in-oxy]-5-fluorphenyl)-2-isopropyl-4-methyl-2,3-dihydrobenzothiazin-3-on-1,1-dioxid und 1-(2-[3,4,5-Trimethoxyphenyl]-ethyl)-piperazin.
Dihydrochlorid: Fp. 160-162 °C
$^1$H-NMR : δ = 7,63-7,7 (m,2H), 7,3-7,4 (m,1H), 6,95-7,03 (m,2H), 6,68-6,80 (m,2H), 6,37 (s,2H), 4,51 (m,2H), 3,79 (s,6H), 3,75 (s,3H), 3,45 (s,3H), 3,23 (m,2H), 3,12 (m,1H), 2,4-2,8 (m,12H), 1,15 (dd,6H)
Das als Ausgangsmaterial verwendete 2-(2-[4-Chlorbut-2-in-oxy]-5-fluorphenyl)-2-isopropyl-4-methyl-2,3-dihydrobenzothiazin-3-on-1,1-dioxid wird wie folgt hergestellt:

b) 8,28 g 2-(2-Hydroxy-5-fluorphenyl)-2-isopropyl-4-methyl-2,3-dihydrobenzothiazin-3-on werden in 400 ml Dichlormethan bei Raumtemperatur mit 13,4 g 3-Chlorperbenzoesäure portionsweise versetzt. Man rührt 2 Stunden nach und wäscht mit 3 x 100 ml gesättigter Natriumbicarbonatlösung. Die organische Phase wird getrocknet und i. Vak. eingeengt. Man erhält das ölige Dioxid.
$^1$H-NMR : δ = 6,4-8,0 (m,8H), 3,48 (s,3H), 1,2 (dd,6H)

c) 9,08 g des in Beispiel 9b erhaltenen rohen Phenol-Derivates werden in 150 ml 2-Butanon und 10,35 g gemahlenem Kaliumcarbonat mit 7,3 ml 1,4-Dichlor-2-butin 5 Stunden unter Rückfluß gerührt. Nach dem Erkalten saugt man den Niederschlag ab und engt das Filtrat i. Vak. ein. Das zurückbleibende Öl wird an $SiO_2$ chromatographisch gereinigt.
(Elutionsmittel Cyclohexan/Essigester 3:1).
$^1$H-NMR : δ = 6,6-7,85 (m,7H), 4,62 (m,2H), 4,16 (m,2H), 3,55 (s,3H), 3,2 (m,1H), 1,23 (dd,6H)

## Beispiel 10

2-[2-[4-[4-[2-(3,4,5-Trimethoxyphenyl)-ethyl]-piperazin-1-yl]-but-2-en-oxy]-5-fluorphenyl]-2-isopropyl-4-methyl-2,3-dihydrobenzothiazin-3-on-1,1-dioxid

2,0 g des in Beispiel 9a beschriebenen Acetylenderivates werden in 10 ml Pyridin gelöst und mit 0,2 g Pd/$BaSO_4$ versetzt. Man hydriert in der Schüttelente, bis die berechnete Menge an Wasserstoff aufgenommen ist. Nach Abtrennen des Katalysators und Verdampfen des Lösungsmittels erhält man ein Rohprodukt, das an 80 g $SiO_2$ mit Dichlormethan/Methanol 98:2 als Elutionsmittel gereinigt wird.
Dihydrochlorid: Fp. 183-185 °C
$^1$H-NMR (Base) : δ = 7,65-7,8 (m,2H), 7,44 (m,1H), 6,97-7,13 (m,2H), 6,78-6,9 (m,1H), 6,64-6.73 (m,1H), 6,45 (s,2H), 5,75 (m,2H), 4,5 (m,2H), 3,87 (s,6H), 3,83 (m,3H), 3,2 (m,1H), 3,05 (m,2H), 20 2,4-2,8 (m), 1,1-1,4 (m,6H)

## Beispiel 11

2-[2-[4-[4-[2-(3,4,5-Trimethoxyphenyl)-ethyl]-piperazin-1-yl]-but-2-in-oxy]-5-fluorphenyl]-7-fluor-2-isopropyl-4-methyl-2,3-dihydrobenzothiazin-3-on-1,1-dioxid

Die Herstellung erfolgt analog Beispiel 9.
Dihydrochlorid: Fp. 206-208 °C
$^1$H-NMR (Base) : δ = 7,7 (m,1H), 7,45 (dd,1H), 7,1 (m,2H), 6,84 (m,2H), 6,43 (s,2H), 4,58 (m,2H), 3,85 (s,6H), 3,82 (s,3H), 3,50 (s,3H), 3,30 (m,2H), 3,17 (m,1H), 2,75 (m,2H), 2,60 (m,10H), 1,26 (d,3H), 1,16 (d,3H)

**Beispiel 12**

2-[2-[4-[4-[2-(3,4,5-Trimethoxyphenyl)-ethyl]-piperazin-1-yl]-but-2-en-oxy]-5-fluorphenyl]-7-fluor-2-isopropyl-4-methyl-2,3-dihydrobenzothiazin-3-on-1,1-dioxid

Die Herstellung erfolgt aus der Verbindung aus Beispiel 11 analog Beispiel 10.
Dihydrochlorid: Fp. 230-232 °C
$^1$H-NMR (Base) : δ = 7,68 (dd,1H), 7,47 (dd,1H), 7,13 (m,1H), 6,98 (dd,1H), 6,86 (m,1H), 6,19 (dd,1H), 6,43 (s,2H), 5,74 (m,2H), 4,48 (m,2H), 3,85 (s,6H), 3,8 (s,3H), 3,43 (s,3H), 3,17 (m,1H), 1,25 (d,3H), 1,13 (d,3H)

**Beispiel 13**

2-[2-[3-[4-[2-(3,4,5-Trimethoxyphenyl)-ethyl]-piperazin-1-yl]-2-hydroxypropoxy]-phenyl]-2-ethyl-4,7-dimethyl-2,3-dihydrobenzothiazin-3-on-1,1-dioxid

Die Herstellung erfolgt aus 2-(2-[2,3-Epoxypropoxy]-phenyl)-2-ethyl-4,7-dimethyl-2,3-dihydrobenzothiazin-3-on-1,1-dioxid und 1-(2-[3,4,5-Trimethoxyphenyl]-ethyl)-piperazin.
$^1$H-NMR (Base) : δ = 6,45-7,95 (m,7H), 6,40 (s,2H), 3,80 (s,6H), 3,76 (s,3H), 3,45 (s,3H), 2,4 (s,3H)

**Beispiel 14**

2-[2-[4-[4-[2-(3,4,5-Trimethoxyphenyl)-ethyl]-piperazin-1-yl]-4-oxobutoxy]-phenyl]-2-cyclohexyl-4-methyl-2,3-dihydrobenzothiazin-3-on-1,1-dioxid

3,2 g 2-(2-[3-Carboxypropoxy]-phenyl)-2-cyclohexyl-4-methyl-2,3-dihydrobenzthiazin-3-on-1,1-dioxid in 15 ml DMF wird mit 1,05 g Hydroxybenztriazol, 2,1 g 1-(2-[3,4,5-Trimethoxyphenyl]-ethyl)-piperazin und 1,85 g Dicyclohexylcarbodiimid 2 Stunden bei Zimmertemperatur gerührt. Man läßt über Nacht stehen, saugt von den Kristallen ab, gießt das Filtat auf 100 ml Eiswasser und extrahiert das Gemisch 3 x mit Ethylacetat. Die vereinigten organischen Phasen werden mit Bicarbonat-Lösung und Wasser gewaschen, getrocknet und eingedampft. Der Rückstand wird an Kieselgel (120 g) mit Methylenchlorid/Methanol 9:1 gereinigt.
$^1$H-NMR : δ = 6,45-8,0 (m,8H), 6,43 (s,2H), 3,81 (s,6H), 3,78 (s,3H), 3,43 (s,3H), 0,9-2,0 (s,13H)

**Beispiel 15**

(+)-Enantiomer von 2-[2-[5-(4-[2-(3,4,5-Trimethoxyphenyl)-ethyl]-piperazin-1-yl)-3-oxapentyloxy]-phenyl]-2-isopropyl-4-methyl-2,3-dihydrobenzothiazin-3-on-1,1-dioxid

a) Herstellung erfolgt analog Beispiel 1a aus (+)-2-(2-[5-Brom-3-oxapentyloxy]-phenyl)-2-isopropyl-4-methyl-2,3-dihydrobenzothiazin-3-on-1,1-dioxid und 1-(2-[3,4,5-Trimethoxyphenyl]-ethyl)-piperazin
$^1$H-NMR : δ = 6,45-7,9 (m,8H), 6,35 (s,2H), 3,80 (s,6H), 3,77 (s,3H), 3,40 (s,3H), 2,9-4,1 (m,7H), 2,3-2,8 (m,14H), 1,17 (dd,6H)
Oxalat: Fp. 209-211 °C (Zers.)

b) Das als Ausgangsmaterial benötigte (+)-2-(2-[5-Brom-3-oxapentyloxy]-phenyl)-2-isopropyl-4-methyl-2,3-dihydrobenzothiazin-3-on-1,1-dioxid wird hergestellt analog Beispiel 1b aus (+)-2-(2-[5-Brom-3-oxapentyloxy]- phenyl)-2-isopropyl-4-methyl-2,3-dihydrobenzothiazin-3-on durch Oxydation mit 3-Chlorperbenzoesäure.

**Beispiel 16**

2-[3-[3-(4-[2-(3,4,5-Trimethoxybenzoyl)-piperazin-1-yl)-propoxy]-phenyl]-2-isopropyl-4-methyl-2,3-dihydrobenzothiazin-3-on-1,1-dioxid

Die Herstellung erfolgt analog Beispiel 1a aus 2-(3-[3-Brompropoxy]-phenyl)-2-isopropyl-4-methyl-2,3-dihydrobenzothiazin-3-on-1,1-dioxid
$^1$H-NMR : δ = 6,5-8,0 (m,8H), 6,65 (s,2H), 3,78 (s,6H), 3,67 (s,3H), 3,40 (s,3H), 1,1 (dd,6H),

**Beispiel 17**

2-[2-[4-(1-[3,4-Dimethoxyphenyl)-2-methyl-2-propylamino]-butoxy]-phenyl]-2,4-dimethyl-2,3-dihydrobenzothiazin-3-on-1-oxid

a) Die Herstellung erfolgt wie in Beispiel 1a beschrieben aus 2-(2-[4-Brombutoxy]-phenyl)-2,4-dimethyl-2,3-dihydrobenzothiazin-3-on-1-oxid und 1-(3,4-Dimethoxyphenyl)-2-methyl-2-propylamin
$^1$H-NMR : δ = 6,5-7,7 (m,11H), 3,75 (s,6H), 3,45 (s,3H), 1,0 (s,6H)
Hydrochlorid: Fp. 215 °C

b) Das als Ausgangsmaterial benötigte Sulfoxid wird folgendermaßen hergestellt:
8,08 g 2-(2-[Brombutoxy]-phenyl)-2,4-dimethyl-2,3-dihydrobenzothiazin-3-on wird in 200 ml Dichlormethan gelöst und bei Zimmertemperatur portionsweise mit 3-Chlorperbenzoesäure versetzt, bis das Ausgangsmaterial vollständig umgesetzt ist (DC-Kontrolle mit Chloroform/Methanol 95:5 als Laufmittel). Es werden etwa 5,3 g 80 bis 90 %ige 3-Chlorperbenzoesäure benötigt. Nach 1-stündigem Nachrühren wird 3x mit gesättigter Bicarbonatlösung gewaschen, die organische Phase getrocknet und i.Vak. eingeengt.

**Beispiel 18**

2-[2-[4-[4-[2-(3,4,5-Trimethoxyphenyl)-ethyl]-piperazin-1-yl]-butoxy]-phenyl]-2-isopropyl-4-methyl-2,3-dihydrobenzothiazin-3-on-1-oxid

Die Herstellung erfolgt analog Beispiel 1a aus 2-(2-[4-Brombutoxy]-phenyl)-2-isopropyl-4-methyl-2,3-dihydrobenzothiazin-3-on-1-oxid (dargestellt analog Beispiel 17b) und 1-(2-[3,4,5-Trimethoxyphenyl]-ethyl)-piperazin
$^1$H-NMR : δ = 6,4-7,6 (m,8H), 6,35 (s,2H), 3,81 (s,6H), 3,77 (s,3H), 3,44 (s,3H), 2,3-2,8 (s,14H), 1,5-2,1 (m,4H), 1,0-1,4 (m,6H)
Dihydrochlorid: Fp. 248-250 °C

**Beispiel 19**

( + )-Enantiomer von 2-[2-[4-[4-(2-(3,4,5-Trimethoxyphenyl)-ethyl]-piperazin-1-yl)-butoxy]-phenyl]-2-isopropyl-4-methyl-2,3-dihydrobenzothiazin-3-on-1-oxid

Die Herstellung erfolgt wie bei der racemischen Verbindung (Beispiel 18), jedoch aus dem ( + )-Enantiomer von 2-(2-[4-Brombutoxy]-phenyl-2-isopropyl-4-methyl-2,3-dihydrobenzothiazin-3-on-1-oxid.
$^1$H-NMR : $\delta$ = 6,5-7,6 (m,8H), 6,40 (s,2H), 3,83 (s,6H), 3,80 (s,3H), 3,45 (s,3H), 1,80 (m,4H), 1,12 (dd,6H)
Dihydrochlorid: Fp. 198-200° C (aus Isopropanol)

**Beispiel 20**

(-)-Enantiomer von 2-[2-[4-[4-[2-(3,4,5-Trimethoxyphenyl)-ethyl]-piperazin-1-yl]-butoxy]-phenyl]-2-isopropyl-4-methyl-2,3-dihydrobenzothiazin-3-on-1-oxid

Die Herstellung erfolgt wie bei der racemischen Verbindung (Beispiel 18), aus dem (-)-Enantiomer von 2-(2-[4-Brombutoxy]-phenyl)-2-isopropyl-4-methyl-2,3-dihydrobenzothiazin-3-on-1-oxid
$^1$H-NMR : $\delta$ = 6,5-7,6 (m,8H), 6,40 (s,2H), 3,8 (s,6H), 3,75 (s,3H), 3,45 (s,3H), 1,75 (m,4H), 1,1 (dd,6H)
Dihydrochlorid: Fp. 215-217° C

**Beispiel 21**

( + )-Enantiomer von 2-[2-[4-[4-[N-Methyl-2-(3,4,5-trimethoxyphenyl)-ethylamino]-piperidin-1-yl]-butoxy]-phenyl]-2-isopropyl-4-methyl-2,3-dihydrobenzothiazin-3-on-1-oxid

Die Herstellung erfolgt analog Beispiel 1a aus ( + )-2-(2-[4-Brombutoxy]-phenyl)-2-isopropyl-4-methyl-2,3-dihydrobenzothiazin-3-on-1-oxid und 4-(N-Methyl-2-[3,4,5-trimethoxyphenyl]-ethylamino)-piperidin
$^1$H-NMR : $\delta$ = 6,4-7,5 (m,8H), 6,35 (s,2H), 3,80 (s,6H), 3,75 (s,3H), 3,43 (s,3H), 1,4-2,2 (m,8H), 0,97-1,4 (dd,6H)
Fp. (Dihydrochlorid): 93-95° C

**Beispiel 22**

2-[2-[4-[4-[2-(3,4,5-Trimethoxyphenyl)-ethyl]-piperazin-1-yl]-butoxy]-phenyl]-2-benzyl-4-methyl-2,3-dihydrobenzothiazin-3-on-1-oxid

Die Herstellung erfolgt analog Beispiel 1a aus 2-(2-[4-Brombutoxy]-phenyl)-2-benzyl-4-methyl-2,3-dihydrobenzothiazin-3-on-1-oxid
$^1$H-NMR : $\delta$ = 6,5-7,6 (m,13H), 6,40 (s,2H), 3,82 (s,6H), 3,77 (s,3H), 3,47 (s,3H), 1,4-1,9 (m,4H),
Oxalat: Fp. 195-7° C (Zers.)

**Beispiel 23**

2-[2-[4-(N-Methyl-2-[3,4-Dimethoxyphenyl)-ethylamino]-butoxy]-phenyl]-2-benzyl-4-methyl-2,3-dihydrobenzothiazin-3-on-1-oxid

Die Herstellung erfolgt analog Beispiel 1a aus 2-(2-[4-Brombutoxy]-phenyl)-2-benzyl-4-methyl-2,3-dihydrobenzothiazin-3-on-1-oxid und N-Methylhomoveratrylamin.
$^1$H-NMR : $\delta$ = 6,3-7,5 (m,11H), 6,75 (s,5H), 8,3 (s,3H), 8,0 (s,3H), 3,47 (s,3H), 1,3-1,8 (m,4H)

**Beispiel 24**

(+)-Enantiomer von 2-(2-[5-(4-[2-(3,4,5-Trimethoxyphenyl)-ethyl]-piperazin-1-yl)-3-oxapentyloxy]-phenyl)-2-isopropyl-4-methyl-2,3-dihydrobenzothiazin-3-on-1-oxid

Herstellung analog Beispiel 1a aus (+)-2-(2-[5-Brom-3-oxapentyloxy]-phenyl)-2-isopropyl-4-methyl-2,3-dihydrobenzothiazin-3-on-1-oxid.
$^1$H-NMR : $\delta$ = 6,4-7,6 (m,8H), 6,35 (s,2H), 3,79 (s,6H), 3,73 (s,3H), 3,35 (s,3H), 2,85-4,1 (s,7H), 1,15 (dd,6H)
Dihydrochlorid: Fp. 111-113 °C

**Beispiel 25**

2-[3-[4-[4-(3,4,5-Trimethoxyphenylacetyl)-piperazin-1-yl]-butoxy]-phenyl]-2-hexyl-4-methyl-2,3-dihydrobenzothiazin-3-on-1-oxid

Die Herstellung erfolgt analog Beispiel 1a aus 2-(3-[4-Brombutoxy]-phenyl)-2-hexyl-4-methyl-2,3-dihydrobenzothiazin-3-on-1-oxid und 4-(3,4,5-Trimethoxyphenylacetyl)-piperazin
$^1$H-NMR : $\delta$ = 6,5-7,6 (m,8H), 6,40 (s,2H), 3,81 (s,6H), 3,77 (s,3H), 3,68 (s,2H), 3,40 (s,3H), 1,0-2,0 (m,12H), 0,85 (t,3H)

**Beispiel 26**

2-[2-[4-(4-[2-(3,4,5-Trimethoxyphenyl)-ethyl]-piperazin-1-yl)-but-2-in-oxy]-5-fluorphenyl]-7-chlor-2-isopropyl-4-methyl-2,3-dihydrobenzothiazin-3-on-1-oxid

Die Herstellung erfolgt analog Beispiel 1a aus 2-(2-[4-Chlorbut-2-in-oxy)-5-fluorphenyl)-7-chlor-2-isopropyl-4-methyl-2,3-dihydrobenzothiazin-3-on-1-oxid.
$^1$H-NMR : $\delta$ = 6,4-7,8 (m,6H), 6,35 (s,2H), 4,60 (m,2H), 3,80 (s,6H), 3,76 (s,3H), 3,45 (s,3H), 1,30 (d,3H), 1,2 (d,2H)

## Ansprüche

1. Benzothiazinon-Oxide der Formel I

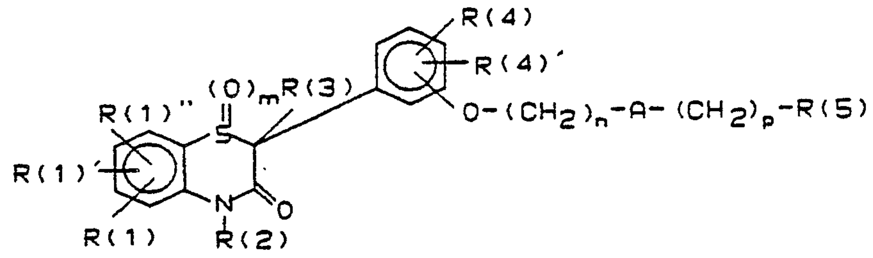

(I)

sowie ihrer Salze mit pharmazeutisch akzeptablen Säuren, dadurch gekennzeichnet, daß die folgenden Substituenten und Indices folgende Bedeutung haben:

R(1), R(1)′ und R(1)″, gleich oder verschieden und voneinander unabhängig, Wasserstoff, $(C_1-C_4)$-Alkyl, $(C_1-C_3)$-Alkoxy, F, Cl, Br, $CF_3$, Nitro, Hydroxy, Acetamido oder Amino,

R(2) Wasserstoff, $(C_1-C_{10})$-Alkyl, geradkettig oder verzweigt, $(C_3-C_{10})$-Alkenyl, geradkettig oder verzweigt, Phenyl-$(C_1-C_4)$-alkyl, wobei der Phenylring unsubstituiert oder durch einen, zwei oder drei Substituenten aus der Gruppe $(C_1-C_4)$-Alkyl, $(C_1-C_3)$-Alkoxy, F, Cl, $CF_3$, $(C_1-C_2)$-Alkylendioxy oder Nitro substituiert ist,

R(3) Wasserstoff, $(C_1-C_{15})$-Alkyl, geradkettig oder verzweigt, $(C_3-C_{15})$-Alkenyl, geradkettig oder verzweigt, $(C_4-C_8)$-Cycloalkyl, $(C_4-C_8)$-Cycloalkyl-$(C_1-C_4)$-alkyl, Phenyl oder Phenyl-$(C_1-C_4)$-alkyl, wobei der Phenylrest unsubstituiert oder durch einen, zwei oder drei Substituenten der Gruppe $(C_1-C_4)$-Alkyl, $(C_1-C_3)$-Alkoxy, F, Cl, $CF_3$, $(C_1-C_2)$-Alkylendioxy oder Nitro substituiert ist,

R(4) und R(4)′, gleich oder verschieden und voneinander unabhängig, Wasserstoff, $(C_1-C_4)$-Alkyl, $(C_1-C_3)$-Alkoxy, F, Cl, $CF_3$, Nitro, Hydroxy, Acetamido oder Amino,

A eine CH(OH)-Gruppe, eine C=O-Gruppe, eine CH=CH-Gruppe, eine C≡C-Gruppe, eine $CH_2$-Gruppe, Sauerstoff oder Schwefel,

m 1 oder 2,

n 1, 2 oder 3,

p Null, 1, 2, 3 oder 4; sofern A ein Heteroatom ist, jedoch nur 2, 3 oder 4; und sofern A eine CH(OH), CH=CH- oder C≡C-Gruppe ist, jedoch nur 1, 2, 3 oder 4,

R(5) eine der folgenden Gruppen

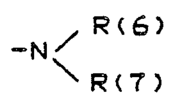

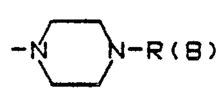

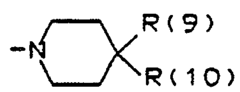

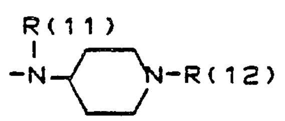

oder

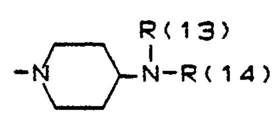

,

worin

R(6) und R(7),

gleich oder verschieden und voneinander unabhängig, Wasserstoff, $(C_1-C_{10})$-Alkyl, $(C_4-C_8)$-Cycloalkyl, $(C_4-C_8)$-Cycloalkyl-$(C_1-C_4)$-alkyl, Pyridyl-$(C_1-C_4)$-alkyl, Phenyl-$(C_1-C_6)$-alkyl, Benzhydryl oder Benzhydryl-$(C_1-C_4)$-alkyl, wobei die Phenylreste jeweils unsubstituiert oder durch einen, zwei oder drei Reste aus der Gruppe $(C_1-C_4)$-Alkyl, $(C_1-C_4)$-Alkoxy, $(C_1-C_2)$-Alkylendioxy, F, Cl, Br, $CF_3$ oder Hydroxy substituiert sind,

R(8)

Wasserstoff, $(C_1-C_{10})$-Alkyl, geradkettig oder verzweigt, $(C_1-C_8)$-Alkanoyl, Pyridyl, Pyrimidinyl, Phenyl, Phenyl-$(C_1-C_4)$-alkyl, Phenyl-$(C_3-C_5)$-alkenyl, Benzhydryl oder Benzyhydryl-$(C_1-C_4)$-alkyl, Phenyl-$(C_1-C_4)$-alkanoyl oder Benzoyl, wobei die Phenylreste jeweils unsubstituiert oder durch einen, zwei oder drei Reste aus der Gruppe $(C_1-C_4)$-Alkyl, $(C_1-C_4)$-Alkoxy, $(C_1-C_2)$-Alkylendioxy, F, Cl, Br, $CF_3$ oder Hydroxy substituiert sind,

R(9)

Wasserstoff, ($C_1$-$C_{10}$)-Alkyl, Phenyl, Phenyl-($C_1$-$C_4$)-alkyl, wobei der Phenylrest jeweils unsubstituiert oder durch einen, zwei oder drei Reste aus der Gruppe ($C_1$-$C_4$)-Alkyl, ($C_1$-$C_4$)-Alkoxy, ($C_1$-$C_2$)-Alkylendioxy, F, Cl, Br, $CF_3$ oder Hydroxy substituiert ist,

R(10)

Wasserstoff, Hydroxy, oder ($C_1$-$C_4$)-Alkoxy, und

R(11) und R(12) bzw. R(13) und R(14),

gleich oder verschieden und voneinander unabhängig Wasserstoff, ($C_1$-$C_{10}$)-Alkyl, geradkettig oder verzweigt, ($C_1$-$C_6$)-Alkanoyl, Phenyl-($C_1$-$C_4$)-alkyl, Benzylhydryl oder Benzhydryl-($C_1$-$C_4$)-alkyl, Phenyl-($C_1$-$C_4$)-alkanoyl, oder Benzoyl, wobei die Phenylreste jeweils unsubstituiert oder durch einen, zwei oder drei Reste aus der Gruppe ($C_1$-$C_4$)-Alkyl, ($C_1$-$C_4$)-Alkoxy, ($C_1$-$C_2$)-Alkylendioxy, F, Cl, Br, $CF_3$ oder Hydroxy substituiert sind, bedeuten.

2. Verbindungen I nach Anspruch 1, dadurch gekennzeichnet, daß mindestens einer der Substituenten oder Indices folgende Bedeutung hat:

R(1) und R(1)′, gleich oder verschieden und voneinander unabhängig, Wasserstoff, Methyl, Ethyl, Methoxy, Ethoxy, Fluor, Chlor, $CF_3$, Nitro oder Acetamido,

R(1)″ Wasserstoff,

R(2) Wasserstoff, ($C_1$-$C_6$)-Alkyl, geradkettig oder verzweigt, Allyl, Methallyl, Benzyl, Phenethyl, 4-Methoxybenzyl, 3,4-Dimethoxybenzyl, 3,4,5-Trimethoxybenzyl, 3,4-Methylendioxybenzyl,

R(3) Wasserstoff, ($C_1$-$C_{12}$)-Alkyl, geradkettig oder verzweigt, Allyl, Methallyl, ($C_5$-$C_7$)-Cycloalkyl, ($C_5$-$C_7$)-Cycloalkyl-($C_1$-$C_4$)-alkyl, Benzyl, Methylbenzyl, Fluorbenzyl, Methoxybenzyl, Dimethoxybenzyl, Phenylethyl,

R(4) Wasserstoff, Methyl, Methoxy, Ethoxy, Fluor, Chlor, Nitro, Hydroxy, Acetamido oder Amino,

R(4)′ Wasserstoff,

A eine CH(OH)-Gruppe, eine C = O-Gruppe, eine CH = CH-Gruppe, eine C≡C-Gruppe, eine $CH_2$-Gruppe, Sauerstoff oder Schwefel,

m 1 oder 2,

n 1 oder 2,

p Null, 1, 2, 3; jedoch, wenn A ein Heteroatom ist, nur 2 oder 3; und, sofern A eine CH(CH), CH = CH oder C≡C-Gruppe ist, nur 1, 2 oder 3,

R(5) eine der folgenden Gruppen

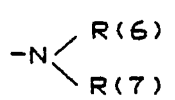
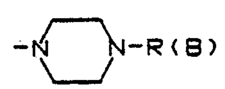
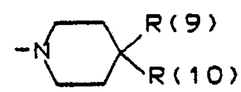

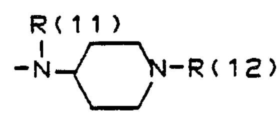
oder
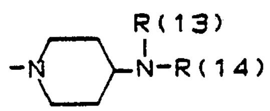,

worin

R(6)

Wasserstoff, Methyl, Ethyl, Propyl, Isopropyl,

R(7)

Wasserstoff, Methyl, Ethyl, Propyl, Isopropyl, Cyclopentylethyl, Cyclohexylethyl, Phenyl-($C_1$-$C_4$)-alkyl, Benzhydryl oder Benzyhydryl-($C_1$-$C_4$)-alkyl, wobei die Phenylreste jeweils unsubstituiert oder durch einen, zwei oder drei Reste aus der Gruppe ($C_1$-$C_4$)-Alkyl, ($C_1$-$C_4$)-Alkoxy, ($C_1$-$C_2$)-Alkylendioxy, F, Cl, $CF_3$ oder Hydroxy substituiert sind, Pyridyl-($C_1$-$C_4$)-alkyl,

R(8)

Wasserstoff, ($C_1$-$C_6$)-Alkyl, geradkettig oder verzweigt, ($C_1$-$C_6$)-Alkanoyl, Phenyl, wobei der Phenylrest durch einen oder zwei Reste aus der Gruppe ($C_1$-$C_4$)-Alkyl, ($C_1$-$C_4$)-Alkoxy, ($C_1$-$C_2$)-Alkylendioxy, F, Cl, $CF_3$ oder Hydroxy substituiert sein kann, Phenyl-($C_1$-$C_4$)-alkyl, Phenyl-($C_3$-$C_5$)-alkenyl, Benzhydryl oder Benzhydryl-($C_1$-$C_4$)-alkyl, Phenyl-($C_1$-$C_4$)-alkanoyl oder Benzoyl, wobei die Reste jeweils substituiert oder durch einen, zwei oder drei Reste aus der Gruppe Methyl, Ethyl, Methoxy, Ethoxy, ($C_1$-$C_2$)-Alkylendioxy, F, Cl $CF_3$ oder Hydroxy substituiert sind,

R(9)

Phenyl, Phenyl-($C_1$-$C_4$)-alkyl, wobei der Phenylrest jeweils unsubstiuiert oder durch einen, zwei oder drei

Reste aus der Gruppe ($C_1$-$C_4$)-Alkyl, ($C_1$-$C_4$)-Alkoxy, ($C_1$-$C_2$)-Alkylendioxy, F, Cl, $CF_3$ oder Hydroxy substituiert ist,

R(10)

Wasserstoff, Hydroxy oder Methoxy

R(11), R(12), R(13) und R(14),

gleich oder verschieden, Wasserstoff, ($C_1$-$C_8$)-Alkyl, ($C_1$-$C_6$)-Alkanoyl, Phenyl-($C_1$-$C_4$)-alkyl, Benzhydryl oder Benzhydryl-($C_1$-$C_4$)-alkyl, Phenyl-($C_1$-$C_4$)-alkanoyl oder Benzoyl, wobei die Phenylreste jeweils unsubstituiert oder durch einen, zwei oder drei Reste aus der Gruppe Methyl, Ethyl, Methoxy, Ethoxy, ($C_1$-$C_2$)-Alkylendioxy, F, Cl, $CF_3$ oder Hydroxy substituiert sind, bedeuten.

3. Verbindungen I nach Anspruch 1, dadurch gekennzeichnet, daß man mindestens einer der Substituenten oder Indices folgende Bedeutung hat:

R(1) Wasserstoff, Methyl, Methoxy, Fluor oder Chlor,

R(1)′ Wasserstoff oder Methoxy,

R(1)″ Wasserstoff,

R(2) Wasserstoff, Methyl, Ethyl, Propyl, Isopropyl, Butyl, sec. Butyl, Isobutyl, Benzyl, Phenethyl,

R(3) Wasserstoff, ($C_1$-$C_{12}$)-Alkyl, geradkettig oder verzweigt, Cyclopentyl, Cyclohexyl, Cyclopentylmethyl, Cyclohexylmethyl, Allyl, Methallyl, Benzyl, Methylbenzyl, Fluorbenzyl, Methoxybenzyl, Dimethoxybenzyl, Phenylethyl,

R(4) Wasserstoff, Methoxy, Methyl, Fluor, Chlor, Nitro oder Hydroxy,

R(4)′ Wasserstoff,

A eine CH(OH)-Gruppe, eine C=O-Gruppe, eine CH=CH-Gruppe, eine C≡C-Gruppe, eine $CH_2$-Gruppe oder Sauerstoff,

m 1 oder 2,

n 1 oder 2,

p Null, 1 oder 2; jedoch, wenn A ein Heteroatom ist, nur 2; und sofern A eine CH(OH), CH=CH oder C≡C-Gruppe ist, nur 1 oder 2,

R(5) eine der folgenden Gruppen

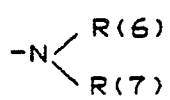 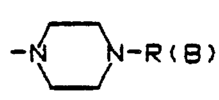 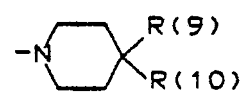

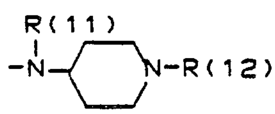 oder 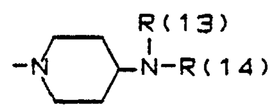 ,

worin

R(6)

Wasserstoff oder Methyl,

R(7)

Phenyl-($C_1$-$C_4$)-alkyl, Benzhydryl oder Benzhydryl-($C_1$-$C_4$)-alkyl, wobei der Phenylrest jeweils unsubstituiert oder durch einen, zwei oder drei Reste aus der Gruppe Methyl, Methoxy, Fluor, Chlor, Methylendioxy oder Hydroxy substituiert ist,

R(8)

($C_1$-$C_6$)-Alkyl, geradkettig oder verzweigt, ($C_1$-$C_6$)-Alkanoyl, Phenyl, Phenyl-($C_1$-$C_4$)-alkyl, Benzhydryl oder Benzhydryl-($C_1$-$C_4$)-alkyl, Phenyl-($C_1$-$C_4$)-alkanoyl oder Benzoyl, wobei die Phenylreste jeweils unsubstituiert oder durch einen, zwei oder drei Reste aus der Gruppe Methyl, Methoxy, Ethoxy, Methylendioxy, Fluor, Chlor, oder Hydroxy substituiert sind,

R(9)

Phenyl, wobei der Phenylrest unsubstituiert oder durch einen, zwei oder drei Reste aus der Gruppe Methyl, Methoxy, Fluor, Chlor, Methylendioxy oder Hydroxy substituiert ist,

R(10)

Wasserstoff, Hydroxy oder Methoxy,

R(11), R(12), R(13) und R(14),

gleich oder verschieden, ($C_1$-$C_6$)-Alkyl, ($C_1$-$C_6$)-Alkanoyl, Phenyl-($C_1$-$C_4$)-alkyl, Benzhydryl oder Benzhydryl-($C_1$-$C_4$)-alkyl, Phenyl-($C_1$-$C_4$)-alkanoyl oder Benzoyl, wobei die Phenylreste jeweils unsubstituiert oder durch

einen, zwei oder drei Reste aus der Gruppe Methyl, Methoxy, Methylendioxy, Fluor, Chlor oder Hydroxy substituiert sind, bedeuten.

4. Verfahren zur Herstellung einer Verbindung I nach Anspruch 1, dadurch gekennzeichnet, daß man

a) eine Verbindung der Formel II

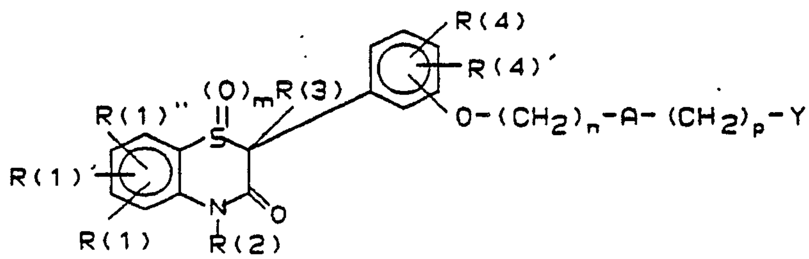

(II)

in welcher R(1), R(1)′, R(1)″, R(2), R(3), R(4), R(4)′, A, m, n und p die gleiche Bedeutung wie in Formel I haben und in welcher Y eine Abgangsgruppe, die nucleophil verdrängt werden kann, mit einer der Verbindungen der Formel IIIa, IIIb, IIIc, IIId oder IIIe

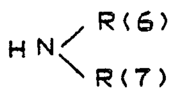

IIIa

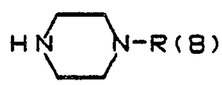

IIIb

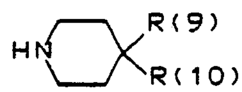

IIIc

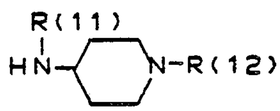

IIId

oder

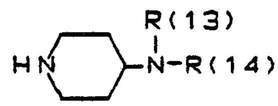

IIIe

in welchen R(6), R(7), R(8), R(9), R(10), R(11), R(12), R(13) und R(14) die gleiche Bedeutung wie in Formel I haben, unter Bedingungen einer nucleophilen Substitution mit oder ohne Gegenwart einer Hilfsbase zum Abfangen der sich bildenden Säure bei einer Temperatur zwischen 0 und 160 °C umsetzt, oder daß man

b) eine Verbindung der Formel IV,

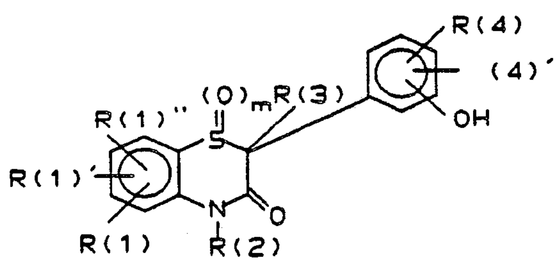

(IV)

in welcher R(1), R(1)′, (R(1)″, R(2), R(3), R(4), R(4)′ und m die gleich Bedeutung wie in Formel I haben, mit einer Verbindung der Formel V,

$Z-(CH_2)_n-A-(CH_2)_p-R(5)$ (V)

in welcher Z gleich wie Y in Formel II definiert ist und in welcher R(5), A, n und p die gleiche Bedeutung wie in Formel I haben, entweder in einem polaren aprotischen Lösungsmittel in Gegenwart einer starken Base bei einer Temperatur zwischen -40 und +60 °C oder in einem protischen oder aprotischen polaren organischen Lösungsmittel in Gegenwart einer schwachen bis mittelstarken Base bei einer Temperatur zwischen 0 und 160 °C umsetzt, oder daß man

c) eine Verbindung der Formel VI

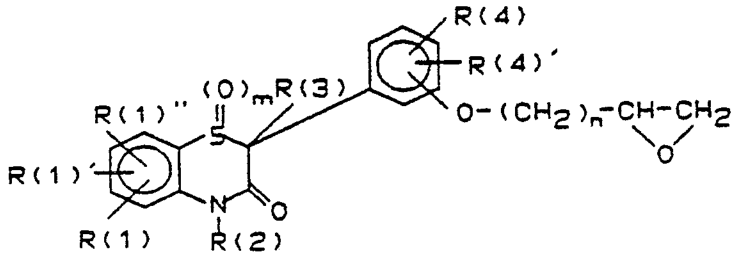

(VI),

in welcher R(1), R(1)′, R(1)″, R(2), R(3), R(4), R(4)′, m und n die gleiche Bedeutung wie in Formel I haben, mit Aminen der Formel IIIa - IIIe ohne Lösungsmittel oder in Gegenwart eines Lösungsmittels umsetzt, wobei Verbindungen der Formel I entstehen, worin A = CH(OH) und p = 1 bedeutet oder daß man

d) eine Verbindung der Formel VII

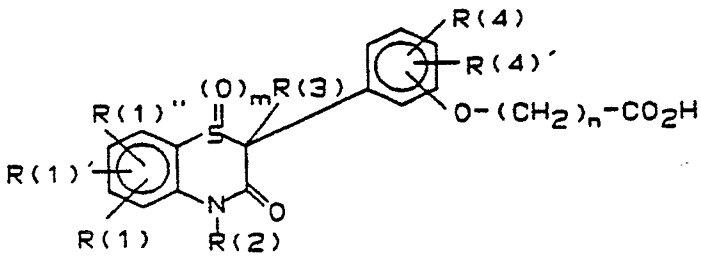

(VII),

in welcher R(1), R(1)′, R(1)″, R(2), R(3), R(4), R(4)′, m und n die gleiche Bedeutung wie in Formel I haben, mit einer der Verbindungen der Formeln IIIa, IIIb, IIIc, IIId oder IIIe unter aus der Literatur bekannten Amidbildungsbedingungen umsetzt, wobei Verbindungen der Formel I entstehen, in denen A eine C=O-Gruppe und p = Null bedeutet,
oder daß man

e) eine Verbindung der Formel VIII

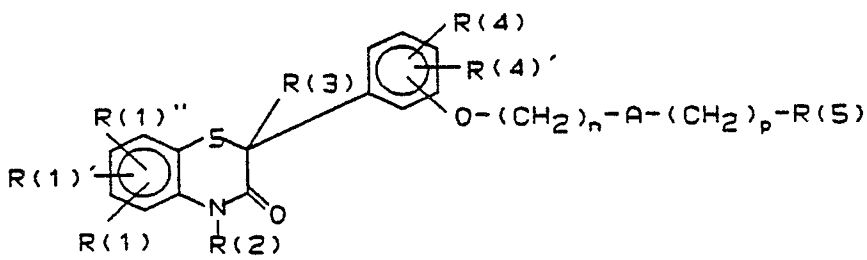

(VIII)

in welcher R(1), R(1)′, R(1)″, R(2), R(3), R(4), R(4)′, R(5), A, n und p die gleiche Bedeutung wie in Formel I haben, mit Oxydationsmitteln zu Verbindungen der Formel I oxidiert.

5. Mittel zur Behandlung von Erkrankungen des Herz-Kreislauf-Systems, welches mindestens eine Verbindung I nach Anspruch 1 und übliche Zusatzstoffe enthält.

6. Verwendung einer Verbindung I nach Anspruch 1 zur Behandlung von Erkrankungen des Herz-Kreislaufsystems.

7. Verwendung einer Verbindung I nach Anspruch 1 zur Herstellung eines Medikaments zur Behandlung von Erkrankungen des Herz-Kreislaufsystems.

Europäisches Patentamt

# EUROPÄISCHER RECHERCHENBERICHT

Nummer der Anmeldung

EP 88 11 2678

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 146 893 (HOECHST AG)<br>* Ansprüche 1-5,10,12,13; & DE - A - 3 347 173 (Kat. D,A) * | 1-7 | C 07 D 279/16<br>C 07 D 417/12<br>A 61 K 31/54 |
| P,A | EP-A-0 243 886 (HOECHST AG)<br>* Ansprüche; & DE - A - 3 614 355 (Kat. P,D,A) * | 1-7 | |
| P,A | EP-A-0 244 723 (HOECHST AG)<br>* Ansprüche; & DE - A - 3 614 363 (Kat. P,D,A) * | 1-7 | |
| A | EP-A-0 116 368 (SANTEN PHARMACEUTICAL CO., LTD.)<br>* Ansprüche 1,22,24,25; Seite 3, Zeilen 20-23 * | 1-7 | |
| P,X | CHEMICAL ABSTRACTS<br>Band 109, Nr. 3, 18. Juli 1988, Seite 32, Abstract Nr. 16764g; J. QAR et al.:"A novel high affinity class of calcium channel blockers"; & MOL. PHARMACOL. 1988, 33(4), 363-9 | 1,5-7 | **RECHERCHIERTE SACHGEBIETE (Int. Cl.4)**<br>C 07 D 279/00 |
| A | GB-A-1 374 283 (E.R. SQUIBB AND SONS)<br>* Ansprüche 1,3; Beispiele 17,18 * | 1 | |

Der vorliegende Recherchenbericht wurde für alle Patentansprüche erstellt

| Recherchenort | Abschlußdatum der Recherche | Prüfer |
|---|---|---|
| BERLIN | 04-11-1988 | HASS C V F |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument